Europäisches Patentamt

European Patent Office

Office européen des brevets

(11) Publication number : **0 518 706 A2**

# (12) EUROPEAN PATENT APPLICATION

(21) Application number : 92305464.7

(22) Date of filing : 15.06.92

(51) Int. Cl.⁵ : **A61K 31/77, // (A61K31/77, 31:765)**

(30) Priority : **13.06.91 JP 167520/91**

(43) Date of publication of application :
**16.12.92 Bulletin 92/51**

(84) Designated Contracting States :
**AT BE CH DE DK FR GB IT LI NL SE**

(71) Applicant : **KOKEN CO., LTD.**
**7, Yonbancho, Chiyoda-ku**
**Tokyo 102 (JP)**

(72) Inventor : **Iijima, Kunihito**
**6-13-11-105 Shimoshakuji, Nerima-ku**
**Tokyo 177 (JP)**
Inventor : **Kato, Haruki**
**140-1 Okawara**
**Hanno-shi, Saitama 357 (JP)**
Inventor : **Nagasu, Yoichiro**
**9-4 Chuo 3-chome**
**Yamato-shi, Kanagawa 242 (JP)**

(74) Representative : **Deans, Michael John Percy et al**
**Lloyd Wise, Tregear & CO. Norman House**
**105-109 Strand**
**London WC2R OAE (GB)**

(54) **An inbihiting agent against proliferation of malignant tumour cells of animals (including human beings)and methods for its manufacture.**

(57)   An inhibiting agent against the proliferation of malignant tumour cells of animals including human beings comprises a mixture of an L-lactic acid straight-chain condensate having a degree of condensation of 5 to 23 and an L-lactic acid cyclic condensate having a degree of condensation of 2 to 15. The agent may be obtained by a process involving first heating lactic acid under normal pressures or reduced pressure in an atmosphere of an inert gas such as nitrogen gas. The thus obtained lactic acid low condensates are dissolved in methanol and filtered. The filtrate is dried under reduced pressure and the residue dissolved in acetonitrile. The resulting solution is passed through a reverse phase system ODS column which has previously been equilibrated by using an acidic acetonitrile solution. A fraction eluted with high concentration acetonitrile solution is collected.

EP 0 518 706 A2

The present invention relates to processes for producing an inhibiting agent against the proliferation of malignant tumour cells of animals, including human beings; and also to the agent so produced.

When malignant tumour cells of animals were cultured, the presence of an active substance that inhibited the proliferation of the malignant tumour cells was detected,

This agent for inhibiting the proliferation of malignant tumour cells was suggested in Japanese Patent Tokkai-sho 59-33223 (Laid-Open No: 33223/1984) and a process for its production was suggested in Japanese Patent Tokkai-sho 60-28930 (Laid-Open No: 28930/1985), but we have found that this process could not be satisfactorily adapted for mass-production of the agent.

When the above active substance was separated, we found that a major component was made up of an L-lactic acid oligomer and a cyclic lower condensate of L-lactic acid.

Although a number of different processes exist which would be capable of producing L-lactic acid lower condensates, since low-molecular-weight oligomers such as the lactic acid lower condensates are very sticky and compatible with each other and have a masking effect, it is difficult to separate the active parts from the reaction product.

The present invention represents a new approach to this problem.

According to a first aspect of the present invention, we provide an inhibiting agent against the proliferation of malignant tumour cells of animals including human beings, comprising a mixture of an L-lactic acid straight-chain condensate having a degree of condensation of 5 to 23 and an L-lactic acid cyclic condensate having a degree of condensation of 2 to 15.

We have devised processes for producing the said inhibiting agent, the present process using reversed phase system column chromatography to separate the active components to produce the aforesaid inhibiting agent.

Accordingly, the present invention provides, in a second and alternative aspect thereof a process for producing an inhibiting agent against the proliferation of malignant tumour cells of animals including human beings, comprising the steps of: heating lactic acid under normal pressures or reduced pressure in an atmosphere of an inert gas, preferably nitrogen; dissolving the thus obtained lactic acid condensates in methanol; filtering the obtained mixture; drying the filtrate under reduced pressure; dissolving the residue in acetonitrile; and passing the resulting solution through a reverse ODS column which has previously been equilibrated by using an acidic acetonitrile solution, thereby collecting a fraction eluted with an acetonitrile solution having a concentration higher than said acetonitrile.

In a modification of this process, in a third alternative aspect of this invention, we provide a process for producing an inhibiting agent against the proliferation of malignant tumour cells of animals including human beings, comprising the steps of: heating lactic acid under normal pressures or reduced pressure in an atmosphere of an inert gas, preferably nitrogen gas; dissolving the thus obtained lactic acid condensates directly in acetonitrile; and passing the resulting solution through a reverse ODS column which has previously been equilibrated by using an acidic acetonitrile solution, thereby collecting a fraction eluted with an acetonitrile solution having a concentration higher than said acetonitrile.

In the case of a product having an approximately single degree of condensation which is obtained by dissolving the reaction solution into acetonitrile and passing the obtained solution through an ODS column to carry out the elution with the concentration gradient being a linear concentration gradient from an aqueous acetonitrile acidic solution having a concentration of 25% to acetonitrile, a large amount of the eluent is required.

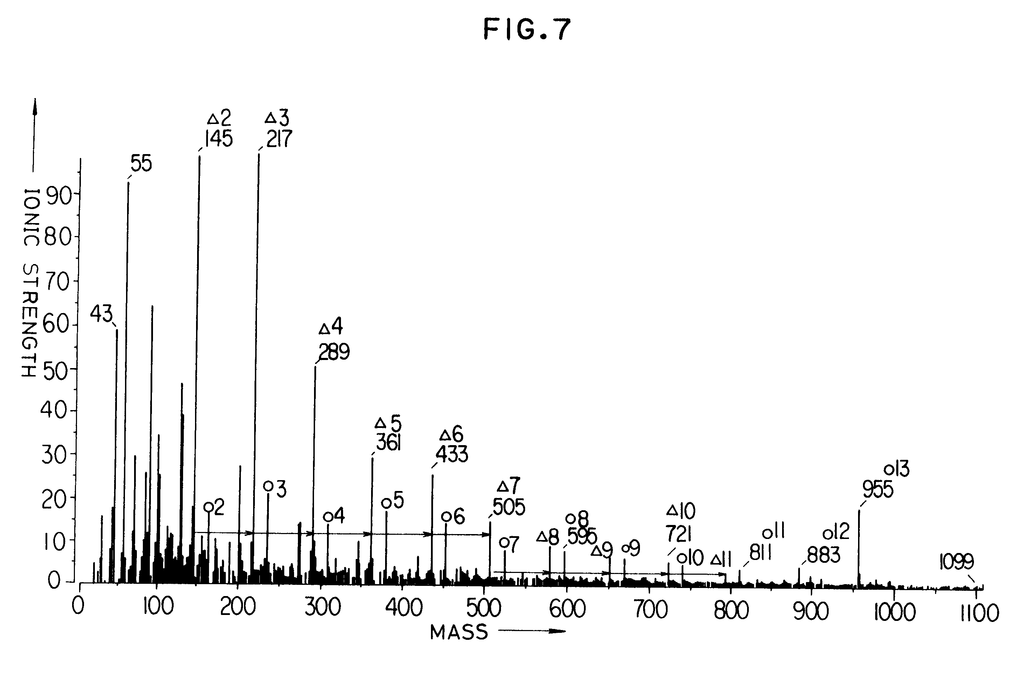

However, as is shown in Fig. 7, in the FAB-MS spectrum of the collected product having a degree of concentration $n = 13$, in addition to the product having a degree of concentration $n = 13$, there are straight-chain condensates and cyclic condensates having a degree of concentration = 13 or less and particularly cyclic condensates having degrees of concentration = 2 and 3 are mixed in large amounts.

The present L-lactic acid condensate refers to a condensate mainly made up of an L-lactic acid oligomer, a little modified low-molecular-weight L-lactic acid condensate, and an L-lactic acid cyclic condensate.

The temperature at which the heating step is carried out under normal pressures is preferably 140°C or over and the temperature at which the heating step is carried out under reduced pressure is preferably 120°C or over. Stepwise elution is carried out with the concentration of acetonitrile increased successively and is accomplished by eluting with an aqueous acetonitrile acidic solution having a concentration of 70 % or more after elution with an aqueous acetonitrile acidic solution having a concentration of 30 to 50 %.

The elution by means of the reverse phase system ODS or DS column is desirably carried out using an aqueous acetonitrile solution acidified with hydrochloric acid to have a pH of 2 to 3.

The distillation of the unreacted L-lactic acid monomer and low-boiling products may be carried out by heating the reaction mixture at a low temperature under normal pressures to distill off the water present in the monomer and the water produced by the condensation reaction, then carrying out the heating and reduction of the pressure in two steps or three steps to bring the pressure to 200 mmHg or below and the temperature to 150 to 170 °C, and finally heating the reaction mixture at 180 to 200 °C and 10 mmHg or below for 1 to 2 hours.

In the above process, L-lactic acid ($\alpha$-hydroxypropionic acid) is a liquid at room temperature and is generally present in a state wherein two molecules are held together by a hydrogen bond and the solution contains 10 to 20 % of lactic anhydride (a condensate of two molecules).

When L-lactic acid is heated, it is easily dehydrated and polymerised to solidify, and the degree of condensation can be controlled by the amount of water present and the reaction temperature. When the water is distilled off, the temperature of the reaction mixture drops because of the loss of heat of vaporization. The distillation of water should therefore be carried out at a rate of vaporization in line with the heating temperature.

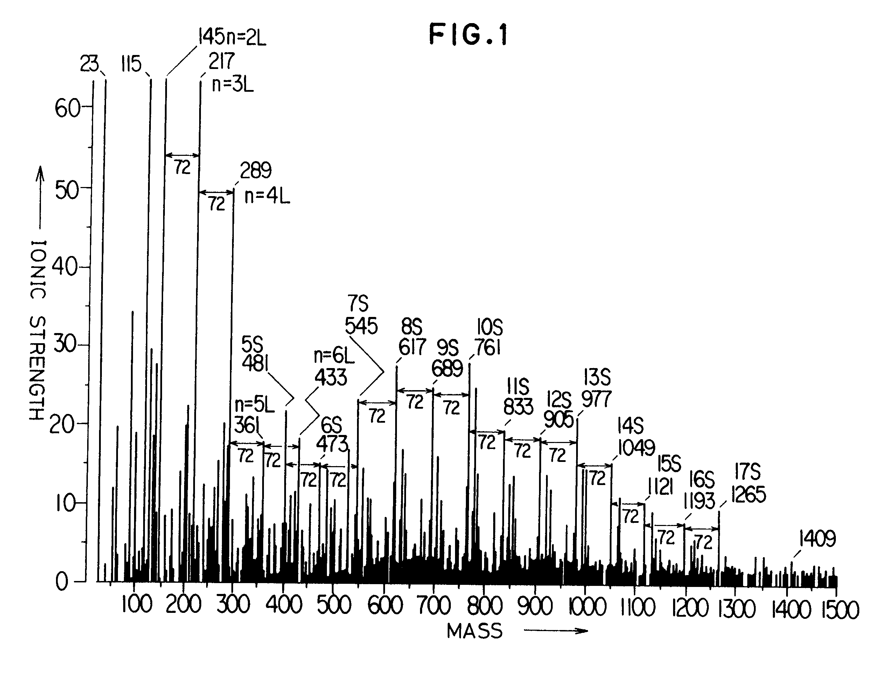

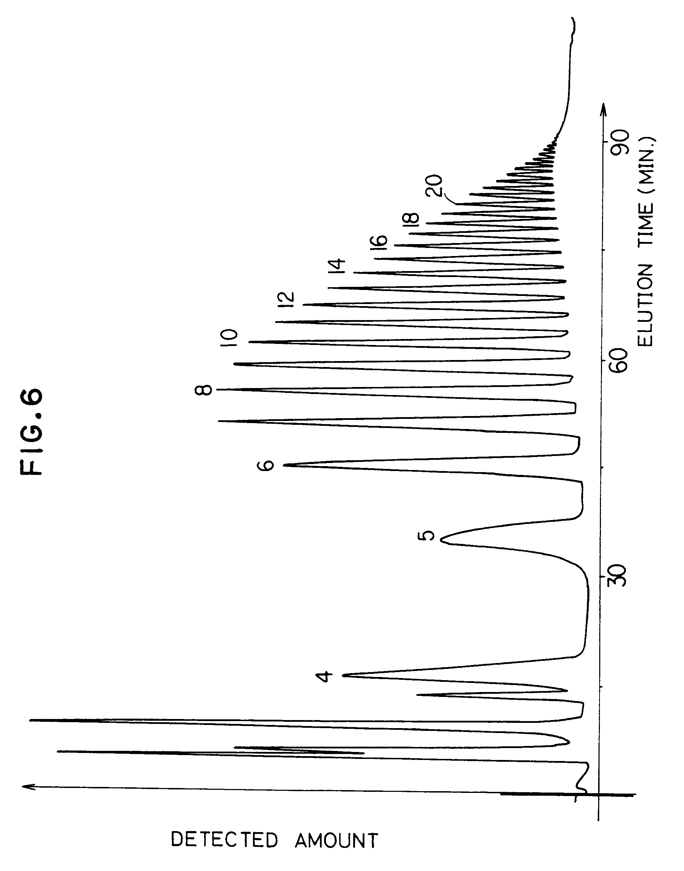

The L-lactic acid oligomer should be one that has a molecular weight of 1,700 or below and can be dissolved or at least can be dispersed into propylene glycol. The lactic oligomer can be obtained, for example, by heating and stirring L-lactic acid at 185 °C under normal pressures for 3 hours while introducing nitrogen gas or also by heating L-lactic acid at a constant temperature of 160 °C with the pressure in the reaction system reduced to 500, 300, 100, and then 50 mmHg stepwise at an interval of 1 to 2 hours. Further, for example, the L-lactic acid oligomer can be obtained by distilling off the coexisting water slowly over about 3 hours under normal pressures at a low temperature, for example, at an oil bath temperature of 145 °C until the content of the L-lactic acid monomer reaches 10 and a few %, then reducing the pressure to 150 mmHg over 0.5 to 1 hour, keeping that pressure for 1.5 to 3 hours, then keeping the reaction mixture for 3 hours at 150 to 160 °C under 10 mmHg or below, and finally continuing the reaction for 1 to 2 hours at 180 to 200 °C. Particularly, the lactic acid low condensate obtained in the last mentioned manner is an oligomer almost free from the monomer and having consecutive degrees of condensation of 3, 4, 5...19, 20.... Further, finally, heating at 180 to 200 °C distills off low-boiling matter. The results are shown in Fig. 1. Generally, as is shown in Fig. 6, although the degree of condensation n varies depending on the reaction conditions, products having degrees of condensation n = 7 or 8 are characteristically most abundant.

In the figure, L stands for lactides of cyclic condensates, So stands for straight-chain condensates, S stands for sodium salts of straight-chain condensates, 2L stands for lactides of cyclic condensates having a degree of condensation n = 2, and 3L stands for lactides of cyclic condensates having a degree of condensation n = 3.

Since the above reaction product solidifies at room temperature and becomes difficult to be redissolved, desirably while the reaction product is still fluid, it is dissolved and dispersed into a certain amount of methanol, or to the reaction product is added ethanol in an amount of 1/5 of the charged amount to form a uniform solution and thereafter methanol is added to the solution to dissolve and disperse the reaction product, or the solidified reaction product is broken by a breaker with a cooler and is stirred in a certain amount of methanol at 50 to 60 °C to be dissolved and dispersed therein, then the dispersion is equilibrated at a temperature of 25 °C, then is filtered and is dried under reduced pressure to obtain an acetonitrile solution, and the solution is fractionated by means of a reverse phase ODS column.

Generally, the reaction liquid is made up of straight-chain condensates and cyclic condensates (lactides), the degree of condensation reaches about 23, it is now impossible to separate them from each other, and although they cannot be identified as a single substance, it appears that they contribute to the inhibition effect in concert though their inhibition effects differ more or less from each other. Although, analytically, cyclic condensates (lactides) having a degree of condensation $\geqq 4$ can be separated from straight-chain condensates roughly, lactides having degrees of condensation = 2 and 3 behave like straight-chain condensates and the former cannot be separated from the latter.

The affinities are very strong and a certain type of reversible equilibrium is established in the closed system. Regarding condensates having a molecular weight of about 1,700 or below which are concerned herein, straight-chain condensates are very soluble in methanol and cyclic condensates are very soluble in acetonitrile.

When the reaction liquid is neutralized and is eluted with a 25 % aqueous acetonitrile solution by means of a reverse phase system ODS column, low-molecular-weight sodium salts are obtained, but cyclic condensates (lactides) cannot be separated unless the eluant contains a large amount of an acetonitrile component.

L-lactic acid ($\alpha$-hydroxypropinonic acid) low condensates are high in cohesion and if the eluant is made acidic to break the hydrogen bonding, they can be separated well by ODS column chromatography.

The invention is hereinafter more particularly described by way of example only, with reference to the accompanying drawings, in which:-

Fig. 1 is an FAB-MS spectrum for a catalyst-free system reaction liquid (neutralized) according to an Example of the invention;

Fig. 2 is an FAB-MS spectrum for the reaction liquid in a case of heating at 185°C under normal pressure for 3 hours according to another Example of the present invention;

Fig. 3 is an FAB-MS spectrum for the reaciton liquid in a case wherein temperature was kept constant at 160°C with pressure decreased from 500 to 300, 100, and then 50 mmHg according to yet another Example of the present invention;

Fig. 4 is an FAB-MS spesctrum for the reaction liquid in a case wherein the reaction was carried out by heating at 160°C under normal pressure for 3 hours and then at 185°C under 3.5 mmHg for 1.5 hours according to a further Example of the present invention;

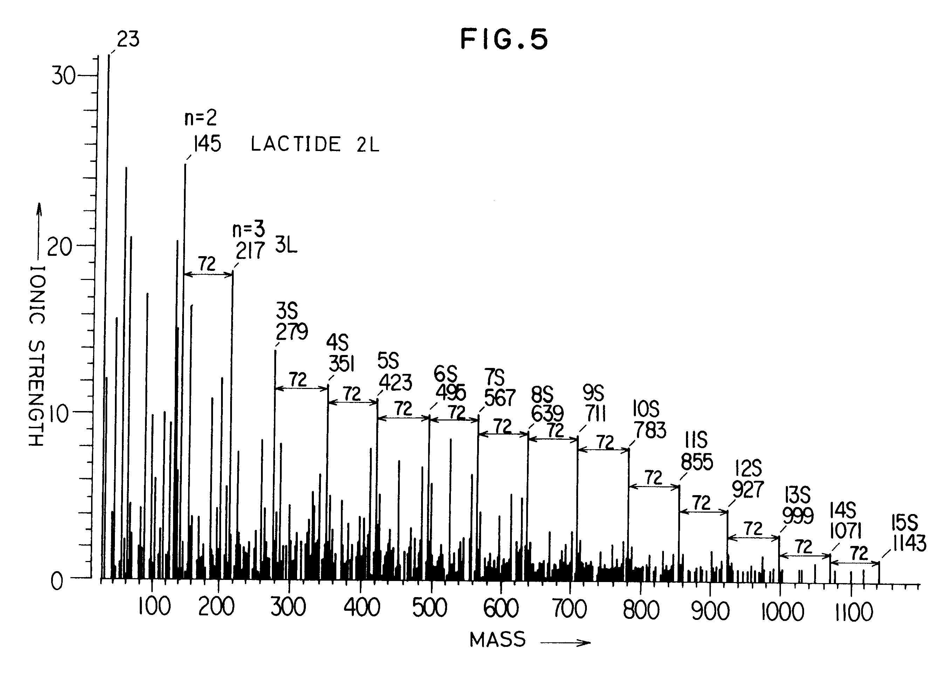

Fig. 5 is an FAB-MS spsectrum of an active part of a catalyst-free system reaction liquid fractionated by means of an ODS column according to a yet further Example of the present invention;

Fig. 6 is a chart of detected concentration/elution time obtained when elution was carried out by an ODS column using a linear concentration gradient from an initial concentration of a 25% aqueous acetonitrile solution (pH: 2.4) to acetonitrile; and

Fig. 7 is an FAB-MS spectrum obtained when the component belonging to the peak of the degree of concentration n = 13 and separated as in the arrangement of Fig. 6 was separated again by chromatography.

## 1. Preparation of L-lactic acid low condensates

(1) 500 ml of L-lactic acid was placed in a separable flask equipped with a descending type connecting tube, a stirring rod, and a nitrogen gas introduction tube, was heated by a heating mantle at an oil bath temperature of 145 °C under normal pressures for 3 hours and then was kept at 145 °C under 150 mmHg for 2 hours. The effluent water was permitted to go through the heat-insulated descending type connecting tube into a flask equipped with a reflux condenser, where it was cooled and held.

Then, the pressure was reduced to a few mmHg over 30 min, and after keeping the temperature at 150 °C for 3 hours, the reaction mixture was heated at 185 °C for 1.5 hours to obtain the intended oligomer (Fig. 1).

Then, while the oligomer was still fluid, it was poured into methanol which was two times as much as the oligomer, and the mixture was cooled on standing at a room temperature of 25 °C and was equilibrated.

(2) The same apparatus as used in (1) was employed and the procedure in (1) was repeated, except that after heating at 155 °C under normal pressures for 3 hours, heating at 160 °C under 40 mmHg for 3 hours was effected, and finally, heating at 185 to 190 oC under a few mmHg for 1.5 hours was effected, thereby obtaining a similar oligomer.

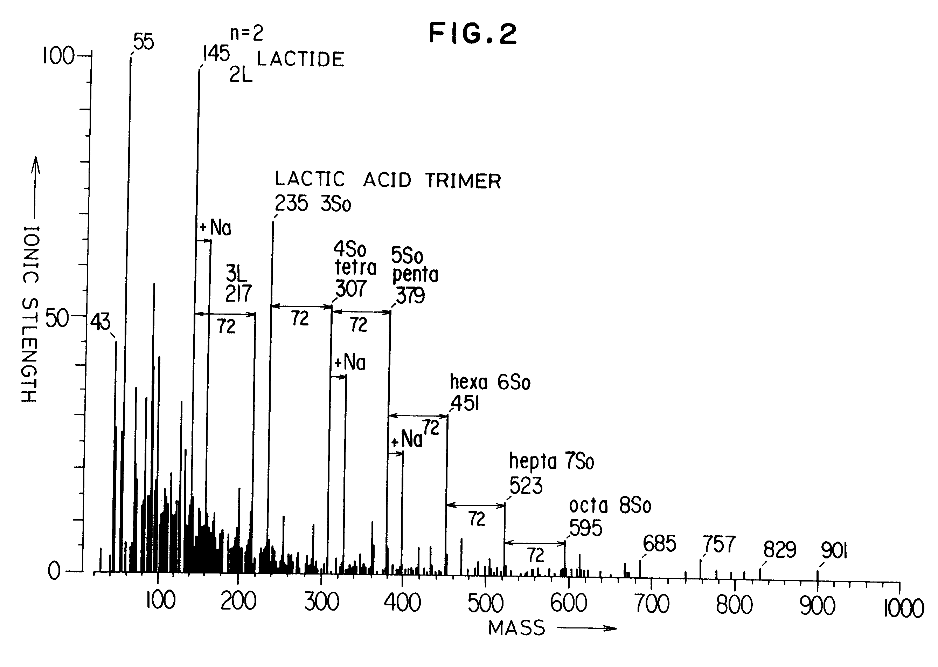

(3) The same apparatus as used in (1) was employed and the procedure in (1) was repeated, except that heating was effected at 185 °C under normal pressures for 3 hours. Low-boiling matter was present (Fig. 2).

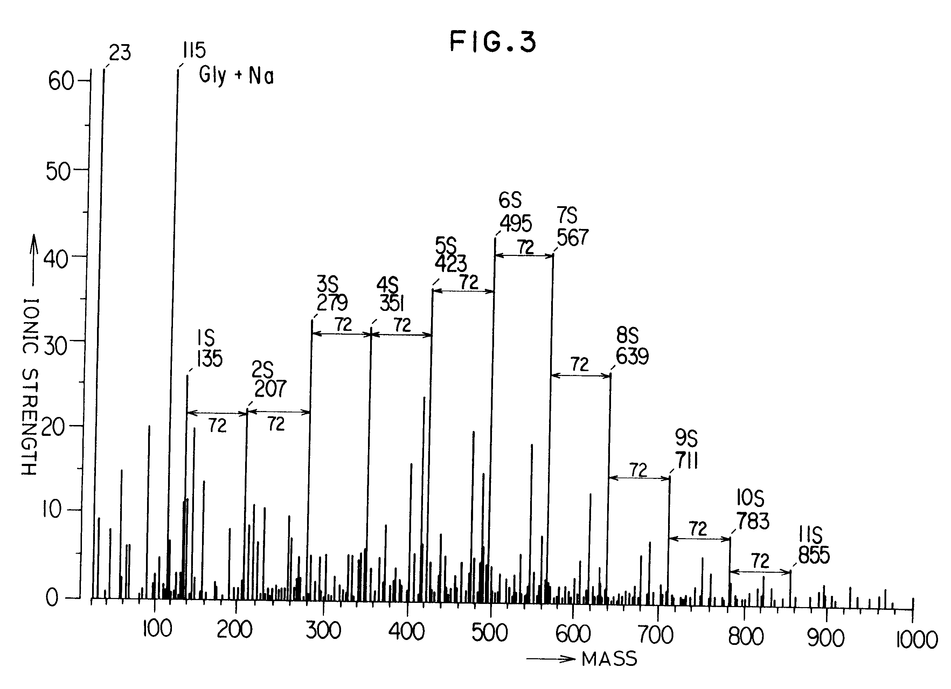

(4) Similarly, the temperature was kept constant to be 160 °C and the degree of vacuum was kept at 500, 300, 100, and then 50 mmHg for every one hour (Fig. 3).

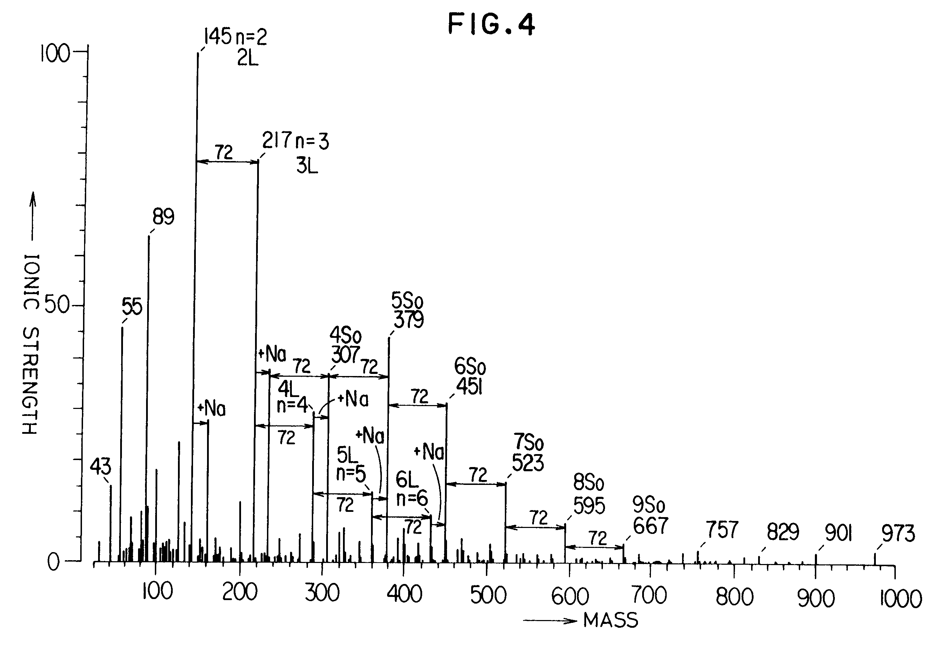

(5) Similarly, after heating at 160 °C under normal pressures for 3 hours, the pressure was reduced to 3.5 mmHg, which was kept at 160 °C, and finally heating was effected at 185 °C for 1.5 hour (Fig. 4).

## 2. Purification of the lactic acid low condensates

(1) The methanol dispersion was filtered at a room temperature of 25 °C through filter paper to obtain methanol-soluble matter, it was dried under reduced pressure to get an acetonitrile solution, and then the acetonitrile solution was passed through a reverse phase ODS (CHEMCO LC - sorbSP - C - ODS) column which had been equilibrated by using a 25 % aqueous acetonitrile acidified with hydrochloric acid to a pH of 2.0 to carry out stepwise elution using hydrochloric acid acidic solutions having acetonitrile concentrations of 25 %, 50 %, and 100 % respectively and a pH of 2.0, thereby obtaining eluted fractions. After neutralizing, they were subjected to ethanol replacement several times, then were dried under reduced pressure, and were dissolved in propylene glycol, naming them Inhibiting Agents 1, 2, and 3 respectively.

(2) Acetonitrile was added to the reaction liquid previously, and was passed through a reverse phase ODS column (ø 1″ x 300 m) which had been equilibrated by using a 25 % aqueous acetonitrile solution(pH: 2.1 to 3.0) and was eluted with the equilibrated aqueous solution for 25 to 30 min at a flow rate of 16.4 ml/min, and the separation was effected using a linear concentration gradient of acetonitrile which was led to acetonitrile over 75 min (Fig. 6).

The above purification resulted in an inhibiting agent made up of an L-lactic acid straight-chain condensate having a degree of condensation of 5 to 23 and an L-lactic acid cyclic condensate having a degree of condensation of 2 to 15.

## 3. Acute toxicity test

(1) Inhibiting Agent 2 obtained in the purification (1) of a lactic acid low condensate was intravenously injected into male mice and the change in weight was observed for 1 week. The results are shown in Table 1.

<u>Table 1</u>

| Dose | Body weight (g) | | |
|---|---|---|---|
| (mg/kg) | After 0 day | After 4 days | After 7 days |
| 250 | 30.4 (died) 30.8 (died) | | |
| 125 | 32.3 30.8 | 32.3 31.0 | 31.8 31.2 |
| 52.5 | 31.4 30.6 | 31.2 30.2 | 31.2 31.2 |
| 31.3 | 32.4 30.4 | 31.9 30.8 | 32.8 30.2 |

(2) Inhibiting Agent 3 obtained in the purification (1) of a lactic acid low condensate was administered intra-arterially to rabbits and after 1 day, 4 days, and 7 days, the body weight was measured. The results are shown in Table 2. For Comparative Example, adriamycin was administered, the results being also shown in Table 2.

<u>Table 2</u>

| Administered agent | Body weight (g) | | |
|---|---|---|---|
| | After 1 day | After 2 days | After 3 days |
| Inhibiting Agent 3 (6 mg/head) | 2.2 2.1 2.1 | 2.1 2.1 2.1 | 2.8 2.1 2.2 |
| Adriamycin (6 mg/head) | 2.2 2.0 2.2 | 2.3 2.0 2.2 | 2.8 died 2.3 |

(3) Inhibiting Agent 2 obtained in the purification (1) of a lactic acid low condensate was administered subcutaneously into the dorsalis of ten 4-week old nude female mice once a day in the course of three days continuously.

Thereafter the progress was observed for 10 days and the result was that the mortality was 0.

(4) Inhibiting Agent 2 obtained in the purification (2) of a lactic acid low condensate was administered to ten 8-week old mice C57B Black through the blood vessel of the tail part once a day in the course of 10 days continuously.

Thereafter, the progress was observed for 10 days and the result was that there was no change in appearance and the mortality was 0.

Dose: 0.2 ml of a 50 mg/ml solution (400 mg/kg)

(5) Inhibiting Agent 2 obtained in the purification (1) of a lactic acid low condensate was intravenously injected (IV) to a 6-month old female Beagle having a body weight of 7 kg once a day continuously, it was observed for 10 days, and the result was that there was no particular abnormal change in appearance (no clinical change).

Dose: 0.7 g (100 mg/kg)

## 4. Experimental Example of inhibition against malignant tumour cells

(1) Human malignant tumor cells (established cells) Hela Cell (human cervical carcinoma established cells) and KB (human fundus oral cavity tumor established cells) were subcutaneously transplanted in the dorsalis of each of nude mice (ICR NU/NU female, 4 weeks old), then from the third day, Inhibiting Agent 1 obtained in the purification (1) of a lactic acid low condensate was administered once a day in the course of 11 days continuously to the experiment group and physiological saline was administered to the control group in a similar manner. Thereafter the administration was stopped and at the seventh week after the transplantation, the tumors were removed and weighed.

The inhibition rate (%) was determined in accordance with the following formula:

$$\left[1 - \frac{\text{weight of tumor of experiment group (g)}}{\text{weight of tumor of control group (g)}}\right] \times 100$$

The results are tabulated in Tables 3 to 4.

Hela Cell (human cervical carcinoma established cells)

Number of transplantation: $1 \times 10^7$

Method of administration: subcutaneous administration (sc)

Dose: 30 mg 0.3 ml (from the third day)

Results: the weight of tumors of 5 cases of each of the control group and the experiment group are shown below.

### Table 3

| No. | Control group (g) | Experiment group (g) |
|-----|-------------------|----------------------|
| 1 | 2.71 | 1.10 |
| 2 | 3.81 | 1.71 |
| 3 | 2.11 | 0.95 |
| 4 | 2.94 | 1.30 |
| 5 | 3.36 | 1.35 |
| $\Sigma x$ | 14.96 | 6.41 |
| x average | 2.99 | 1.28 |

Inhibition rate: 57.1 %

(ii) KB (human fundus oral cavity tumor established cells)

## Table 4

| No. | Control group (g) | Experiment group (g) |
|---|---|---|
| 1 | 6.64 | 2.37 |
| 2 | 6.33 | 3.32 |
| 3 | 6.19 | 1.91 |
| 4 | 6.02 | 2.23 |
| 5 | 6.78 | 3.04 |
| $\Sigma x$ | 31.96 | 12.87 |
| x average | 6.39 | 2.57 |

Inhibition rate: 59.8 %

(2) Mouse lung cancer cells 1.0 x 106 of LLC (mouse lung cancer cells) were transplanted (SC) to each of mice (C57B Black males, 8 weeks old) and from the next day after the transplantation, administration was carried out in the course of 10 days in the same way as in (1). The results are shown in Table 5.

## Table 5

| No. | Control group (g) | Experiment group (g) |
|---|---|---|
| 1 | 3.95 | 2.44 |
| 2 | 4.20 | 1.87 |
| 3 | 4.56 | 2.24 |
| 4 | 3.20 | 2.40 |
| 5 | 4.48 | 2.10 |
| $\Sigma x$ | 20.39 | 10.25 |
| x average | 4.08 | 2.05 |

Inhibition rate: 49.8 %

(3) Yoshida sarcoma

After Yoshida sarcoma was transplanted into each of rats to form a tumor, Inhibiting Agent 2 obtained in the purification (1) of a lactic acid low condensate was administered in amounts of 20 mg/kg and 100 mg/kg intravenously for 7 days continuously and the size (mm) of the tumor was measured. The results are shown in Table 6. Physiological saline was administered to the control group in the same amount. Further, the results obtained using adriamycin as a positive control agent are also shown.

Table 6

| Group | Animal No. | Size of Yoshida sarcoma (mm³)/day | | | |
|---|---|---|---|---|---|
| | | 2 | 3 | 5 | 7 |
| Control group | 1 | 1512 | 3150 | 6650 | 13440 |
| | 2 | 50 | 98 | 600 | 3456 |
| | 3 | 50 | 228 | 1792 | 6500 |
| | 4 | 18 | 40 | 352 | 858 |
| | 5 | 126 | 180 | 1638 | 3795 |
| | 6 | 660 | 770 | 6804 | 12768 |
| Mean ± S.D. | | 395 | 744 | 2973 | 6803 |
| Inhibiting Agent 2: 2 mmg/kg | 16 | 520 | 924 | 3600 | 5382 |
| | 17 | 1092 | 1390 | 3078 | 5616 |
| | 18 | 147 | 256 | 3360 | 4992 |
| Mean ± S.D. | | 586 | 857 | 2346 | 5330 |
| Inhibiting Agent 2: 10 mmg/kg | 19 | 624 | 1560 | 3300 | 5525 |
| | 20 | 108 | 147 | 256 | 630 |
| | 21 | 224 | 450 | 1755 | 3366 |
| | 22 | 1183 | 1547 | 3927 | 5434 |
| | 23 | 416 | 960 | 4522 | 2394 |
| | 24 | 528 | 1014 | 1456 | 1786 |
| Mean ± S.D. | | 514 | 930 | 2536 | 3189 |

Table 6 (continued)

| Group | Animal No. | Size of Yoshida sarcoma (mm$^3$)/day | | | |
|---|---|---|---|---|---|
| | | 2 | 3 | 5 | 7 |
| Adriamycin: 2 mmg/kg | 34 | 320 | 972 | 910 | 576 |
| | 35 | 258 | 864 | 540 | 360 |
| | 36 | 2448 | 4840 | 3960 | 4320 |
| | 37 | 324 | 864 | 720 | 920 |
| | 38 | 308 | 1368 | 1050 | 986 |
| | 39 | 105 | 648 | 384 | 432 |
| | Mean ± S.D. | 627 | 1593 | 1261 | 1265 |

(4) Established cells VX2 originated from rabbit hepatoma

Established cells VX2 originated from rabbit hepatoma were transplanted to each rabbit liver, after two weeks, rabbits with tumors having a size of 10 x 10 mm to 20 x 20 mm were selected, then Inhibiting Agents 2 and 3 in the purification (1) of a lactic acid low condensate were administered intra-arterially and adriamycin was administered intra-arterially to the comparative control. After seven days, the tumors were taken out and observed. The results are shown in Table 7.

EP 0 518 706 A2

## Table 7

| Group | Animal No. | Size of VX 2 cancer $(mm^3)$ | | State of destruction | |
|---|---|---|---|---|---|
| | | Before administration | After administration | VX2 cancer | Normal liver |
| Inhibiting Agent 2: 6 mg/head | 16 | 270 | 478 | The central part was destructed. | NR |
| | 17 | 225 | 320 | The central part was destructed. | NR |
| | 18 | 270 | 336 | Nearly destructed. | NR |
| | Mean ± S.D. | 255 | 377 | | |
| Inhibiting Agent 3: 6 mg/head | 19 | 150 | 120 | Completely destructed. | NR |
| | 20 | 270 | 104 | Completely destructed | Destructed (16 $mm^3$) |
| | 21 | 340 | 264 | Completely destructed. | NR |
| | Mean ± S.D. | 253 | 162 | | |

Table 7 (continued)

| Group | Animal No. | Size of VX 2 cancer (mm³) | | State of destruction | |
|---|---|---|---|---|---|
| | | Before administration | After administration | VX2 cancer | Normal liver |
| Adriamycin: 6 mg/head | 25 | 180 | 180 | Bled to death | NR |
| | 26 | 270 | dead | - | - |
| | 27 | 270 | 330 | The central part was destructed. | NR |
| | Mean ± S.D. | 240 | - | | |

As is shown above, with respect to the size of the cancer (= the longer diameter of the cancer x the shorter diameter thereof), it was observed that there was barely a significant difference in the case of mice, whereas the larger the animals (i.e., in the order of rats and then rabbits) were, the more remarkably the pharmacological effect was exhibited.

## 5. Clinical Examples

Method: A solution of 100 mg of Inhibiting Agent 3 obtained in the purification (1) of a lactic acid low condensate in 1 ml of propylene glycol (which solution is referred to as an inhibiting agent solution hereinafter) was prepared, 30 mg of the Inhibiting Agent (i.e., 0.3 ml of the inhibiting agent solution) per kg of a body weight was mixed with a drops such as a vitamin agent and glucose drops, and the mixture was intravenously instilled. The administration was performed once a day in the course of 5 days continuously, then was suspended for 3 days, and then was resumed for five days, and thereafter the state of the patient was observed with the lapse of time.

## Example 1: Gastric Cancer

When 15 ml of the inhibiting agent solution was mixed with 500 ml of a glucose drops and the mixture was administered in the above stated manner to a patient (60 years old, male) who had a tumor of about the size of a henn's egg, the beneficial effect was exhibited on the fifth day, the bleeding had stopped, his appetite had improved, and his physical condition had been restored. The beneficial effect was followed by roentgenography and a reduction of the tumor was observed. That was also confirmed by a photogastroscope.

## Example 2: Thyroidea Cancer

15 ml of the inhibiting agent solution was mixed with 500 ml of a glucose drops and the mixture was intravenously instilled similarly to a patient (50 years old, male) who had a grown infiltrating tumor and whose blood infiltrated to cause him to have metastasis in lymph nodes. On the sixth day after the administration, the humectation of the blood, etc. had stopped, and with the lapse of time the size of the grown tumor and the lymph of nodes reduced. At the same time, his physical strength had been restored, which is an indication of sufficient significance of the beneficial effect.

## Example 3: Lung Cancer

15 ml of the inhibiting agent solution was mixed with 500 ml of a glucose drops and the mixture was administered similarly to a patient (55 years old, male) who had a grown bronchial cancer. On the fifth day after the administration, the effect was exhibited, the blood in the sputum had disappeared, the cancer cells had reduced remarkably, and an improvement in the whole condition was observed. After two months, roentgenography found that the tumor reduced and it was observed that his physical strength had been remarkably restored.

## Example 4: Uterine Cancer

12 ml of the inhibiting agent solution was mixed with 500 ml of a glucose drops and the mixture was administered similarly to a patient who was suffering from cervical cancer with bleeding. On the fourth or fifth day after the administration, the bleeding had stopped, an improvement in the symptom was observed, and even after one month, bleeding did not occur. After two months, a reduction of the tumor was confirmed by CT scan.

The active part (ODS-80) fractionated by an ODS column contains cyclic condensates together with straight-chain condensates and their degrees of condensation n are continuous to reach high values (a practical degree of condensation $n \leqq 23$), and when the active part is administered, the inhibiting effect begins to appear after the passage of several days after the administration, and it exhibits its effect for so long a period as even if the administration is suspended after ten administrations, the propagation of the tumor is not observed. Therefore, in the case of ODS-80, such administration is considered sufficient that for initial five days the administration is effected once a day and thereafter the administration is effected every 4 or 5 days, but this cannot be confirmed in the case of a mouse because the cycle of metabolism is different.

It is confirmed by many research workers that condensates of $\alpha$-hydroxy acids, in particular condensates of lactic acid, are readily decomposed in the organism and are discharged out of the body and in the flesh always there is lactic acid resulted from fatigue. The decomposition rate of condensates of lactic acid is not so high as to cause lactic acid acidosis, so that an effective amount thereof can be administered successively without pharmacological toxicity, and it is not required to take any side effect into consideration. Further, any species specificity for tumors is not observed.

12

As is described in the above Examples, the present inhibiting agent against malignant tumor cells of animals including human beings caused no particular abnormality in the organism in the toxicity experiments wherein it was used in living organism systems. In the test of inhibition against the proliferation of malignant tumor cells transplanted in mice, the proliferation stopped after several days from the start of the administration and even though the administration was suspended thereafter, it was observed that the tumor significantly reduced, and further in the clinical Examples it was observed that the physical strength of a patient recovered and the tumor reduced. Further, since the subcutaneous administration exhibits the effect stably, the present inhibiting agent is a substance which has a stable prolonged effect in living organisms and is suitable as a carcinostatic agent which is effective and can be used without anxiety.

The present process for producing an inhibiting agent against malignant tumor cells of animals including human beings comprises a chemical process without using any ecosystem and, in comparison with the conventional process, the present process is extremely simplified, the time required can be shortened, and the material cost can be saved.

## Claims

1. An inhibiting agent against the proliferation of malignant tumour cells of animals including human beings, comprising a mixture of an L-lactic acid straight-chain condensate having a degree of condensation of 5 to 23 and an L-lactic acid cyclic condensate having a degree of condensation of 2 to 15.

2. A process for producing an inhibiting agent against the proliferation of malignant tumour cells of animals including human beings, comprising the steps of: heating lactic acid under normal pressures or reduced pressure in an atmosphere of an inert gas, preferably nitrogen; dissolving the thus obtained lactic acid condensates in methanol; filtering the obtained mixture; drying the filtrate under reduced pressure; dissolving the residue in acetonitrile; and passing the resulting solution through a reverse ODS column which has previously been equilibrated by using an acidic acetonitrile solution, thereby collecting a fraction eluted with an acetonitrile solution having a concentration higher than said acetonitrile.

3. A process for producing an inhibiting agent against the proliferation of malignant tumour cells of animals including human beings, comprising the steps of: heating lactic acid under normal pressures or reduced pressure in an atmosphere of an inert gas, preferably nitrogen gas; dissolving the thus obtained lactic acid condensates directly in acetonitrile; and passing the resulting solution through a reverse ODS column which has previously been equilibrated by using an acidic acetonitrile solution, thereby collecting a fraction eluted with an acetonitrile solution having a concentration higher than said acetonitrile.

4. A process according to Claims 2 or 3, wherein said lactic acid is L-lactic acid.

5. A process according to Claims 2 or 3, wherein said lactic acid condensates are L-lactic acid low condensates.

6. A process according to any of Claims 2 to 5, wherein said equilibrating acetonitrile acidic solution has a pH of 2 to 3 and an acetonitrile concentration of 10 to 25%.

7. A process according to any of Claims 2 to 6, wherein the acetonitrile concentration of said acetonitrile acidic solution is increased successively to carry out stepwise elution.

8. A process according to any of Claims 2 to 7, wherein elution is performed with acetonitrile acidic solution having a concentration of 70% or more; and wherein, prior to such elution step, the reverse phase system column is subjected to elution with a 30 to 50% acetonitrile acidic solution having a pH of 2 to 3.

9. A process according to any of Claims 2 to 8, wherein the heating step is carried out under normal pressures at 140°C or over.

10. A process according to any of Claims 2 to 8, wherein the heating step is carried out under reduced pressure at 120°C or over.

11. A process according to Claim 2, wherein the heating step is carried out by first distilling off water present in the monomer and the moisture produced in the condensation reaction by heating at a low temperature under reduced pressures, thereafter reducing the pressure and elevating the temperature in 2 or 3 steps to bring the pressure to 20 mmHg or below and to bring the temperature to 150 to 170°C, and finally heating under 10 mmHg at 180 to 200oC for 1 to 2 hours to distil off the remaining monomer and low-boiling point matter.

FIG.1

FIG.2

EP 0 518 706 A2

FIG.3

EP 0 518 706 A2

FIG.4

EP 0 518 706 A2

FIG.5

FIG.6

EP 0 518 706 A2

# FIG.7

EP 0 518 706 A2